# EUROPÄISCHE PATENTSCHRIFT

(11) **EP 1 044 007 B1**
(45) Veröffentlichungstag und Bekanntmachung des Hinweises auf die Patenterteilung: **05.11.2003**
(21) Anmeldenummer: 98965881.0
(22) Anmeldetag: 29.12.1998
(51) Int. Cl.: A61K 31/7004, A61P 3/00

(54) **MANNOSE ZUR BEKÄMPFUNG DER PROTEINVERLUST-ENTEROPATHIE**
MANNOSE FOR COMBATING PROTEIN LOSS ENTEROPATHY
LE MANNOSE POUR LUTTER CONTRE L'ENTEROPATHIE PAR PERTE DE PROTEINES

(30) Priorität: 29.12.1997 DE 19758059
(43) Veröffentlichungstag der Anmeldung: 18.10.2000
(73) Patentinhaber: SHS Gesellschaft für Klinische Ernährung mbH, 74074 Heilbronn (DE); Marquardt, Thorsten, 48149 Münster (DE)
(72) Erfinder: MARQUARDT, Thorsten, D-48149 Münster (DE)
(74) Vertreter: Köster, Hajo, Dr.
(86) Internationale Anmeldenummer: EP9808508
(87) Internationale Veröffentlichungsnummer: WO99033474

(56) Entgegenhaltungen:
- EP-A- 0 555 980
- GB-A- 2 265 310
- FREEZE H.H.: "disorders in protein glycosylation and potential therapy: tip of an iceberg?" J OF PEDIATRICS, Bd. 133, Nr. 5, November 1998, Seiten 593-600, XP002103393
- NIEHUES ET AL: "carbohydrate-deficient glycoprotein sydrome type IB" J OF CLINICAL INVEST., Bd. 101, Nr. 7, 1. April 1998, Seiten 1414-1420, XP002103394

## Beschreibung

Die Erfindung betrifft die Verwendung von Mannose zur Bekämpfung der Proteinverlust-Enteropathie bzw. des Phosphomannose Isomerase-Defektes und eine mannosehaltige Darreichungsform.

Die Proteinverlust-Enteropathie stellt eine schwerwiegende Erkrankung dar, die sich durch chronische Durchfälle, Eiweißverlust und Gedeihstörung auszeichnet. Sie wird häufig begleitet von Thrombosen und lebensbedrohenden Blutungen. Unter Umständen führt diese Erkrankung in schweren Fällen zu einem vorzeitigen Versterben der betroffenen Patienten.

Aufgabe der vorliegenden Erfindung ist es, einen Weg zur Bekämpfung dieser Erkrankung aufzuzeigen.

Gelöst wird diese Aufgabe durch die Lehre der Ansprüche.

Durch die Verabreichung von Mannose kann die Proteinverlust-Enteropathie somit therapiert werden. Diese Therapie führt zu einer kompletten Gesundung. Somit verschwinden alle klinischen Symptome.

Es gibt eine Vielzahl von Publikationen, die sich mit dem Krankheitsbild der Proteinverlust-Enteropathie befassen. Bis jetzt konnte jedoch noch nicht aufgeklärt werden, durch welche Faktoren bzw. Ursachen diese Erkrankung hervorgerufen wird.

Es wurde nun überraschend gefunden, daß die Ursache der Proteinverlust-Enteropathie in einem vererbten, d.h. genetisch bedingten, Fehler in einem wichtigen körpereigenen Eiweiß, nämlich der Phosphomannose Isomerase, besteht. Letzteres Eiweiß spielt im Stoffwechsel der Mannose eine sehr wichtige Rolle. Bei der Mannose handelt es sich bekanntlich um einen Zucker, der mit dem Traubenzucker chemisch verwandt ist.

Fast alle Eiweißstoffe im Körper brauchen für ihre Funktion bestimmte Zuckerreste. Der Hauptbestandteil dieser Zuckerreste ist die Mannose. Aufgrund des Defektes in der Phosphomannose Isomerase steht bei der Proteinverlust-Gastroenteropathie für die Zuckerreste keine ausreichende Menge an Mannose zur Verfügung, so daß der Aufbau sehr vieler Eiweiße im Körper gestört ist. Als Folge entsteht dabei eine Reihe von Symptomen, bei denen die Proteinverlust-Gastroenteropathie im Vordergrund steht.

Im Blut kann man diese Störung der Zuckerreste an den Eiweißstoffen mit Hilfe einer speziellen Untersuchungstechnik, nämlich der sogenannten isoelektrischen Fokussierung, nachweisen. Störungen des Zuckeranteils in den Bluteiweißen werden im internationalen Schrifttum gewöhnlich als CDG-Syndrom (Carbohydrate deficient glycoprotein) bezeichnet. Da die Proteinverlust-Enteropathie auch auf einer Störung des Zuckeranteils in den Bluteiweißen beruht, wird vorgeschlagen, diese Stoffwechselerkrankung als CDG-Syndrom Ib zu bezeichnen. Die Bezeichnung Ib wurde deshalb gewählt, weil die Blutveränderungen dem CDG-Syndrom Typ la stark ähneln. Dabei ist allerdings zu betonen, daß das CDG-Syndrom Typ Ib, nämlich der Phosphomannose Isomerase-Defekt, trotz der Namensähnlichkeit ansonsten mit den anderen CDG-Syndromen nichts zu tun hat. Es handelt sich dabei um eine völlig andere Erkrankung mit ebenfalls völlig anderen Symptomen. Alle bekannten CDG-Syndrome vom Typ la zeichnen sich durch eine sehr schwere geistige und motorische Behinderung aus. Bei der Proteinverlust-Enteropathie bzw. bei dem Phosphomannose Isomerase-Defekt handelt es sich hingegen um eine Erkrankung des Magen-Darm-Traktes und der Leber.

Durch die Verabreichung von Mannose wird der erblich bedingte Stoffwechselblock umgangen. Diese Therapie muß lebenslang erfolgen.

Gegenstand der Erfindung ist somit die Verwendung von Mannose zur Herstellung eines Mittels zur Bekämpfung der Proteinverlust-Enteropathie bzw. zur Behandlung des Phosphomannose Isomerase-Defektes. Die Mannose wird dabei vorzugsweise in einer Menge von 10 bis 1000 mg/kg Körpergewicht und pro Tag verabreicht. Vorzugsweise wird die Mannose in einer Dosierung von 50 bis 200 mg/kg Körpergewicht 3-5 mal am Tag (insbesondere bevorzugt 5 x 150 mg/kg) verabreicht.

Zur Durchführung dieser Therapie kann man beispielsweise eine Darreichungsform herstellen, welche Mannose enthält. Erforderlichenfalls können übliche Träger und Excipienten vorhanden sein. Diese Darreichungsform kann auch ausschließlich aus Mannose bestehen.

Nach einer bevorzugten Ausführungsform stellt die erfindungsgemäße Darreichungsform eine Einzeldosis dar und enthält 0,2 bis 30 g Mannose und insbesondere 2 bis 20 g Mannose.

Die Mannose kann oral verabreicht werden und kann beispielsweise nach Auflösen in einer Flüssigkeit, beispielsweise Wasser, vom Patienten getrunken werden. Daher besteht eine erfindungsgemäße Ausführungsform der erfindungsgemäßen Darreichungsform auch aus einer wäßrigen Mannoselösung.

Bei der erfindungsgemäßen Darreichungsform kann es sich dabei um ein pharmazeutisches Mittel, welches die Mannose vorzugsweise als Wirkstoff enthält, oder um ein Nahrungsmittel beliebiger Art handeln. Die Mannose kann somit auch einem Nahrungsmittel einverleibt sein.

Statt der reinen Mannose können erfindungsgemäß auch mannosehaltige Zucker, beispielsweise Disaccharide und Oligosaccharide, eingesetzt werden, aus denen im Magen-Darm-Trakt Mannose freigesetzt wird, beipielsweise enzymatisch. Die oben angegebenen, zu verabreichenden Mengen an Mannose beziehen sich dann auf die Mannoseeinheit(en) dieser Zucker.

Es können auch Mischungen aus zweier oder mehreren derartigen mannosehaltigen Zuckern oder aus reiner Mannose und einem oder mehreren derartigen mannosehaltigen Zucker(n) verabreicht werden.

Wenn im Rahmen der vorliegenden Unterlagen von Bereichen, beispielsweise Mengenbereichen, die Rede ist, dann sind auch alle zwischen den Bereichsgrenzen liegenden Zwischenwerte, insbesondere ganzzahlige Zwischenwerte, und ferner auch eingere Bereichsgrenzen umfasst und offenbart. So steht beipielsweise der Bereich 10 bis 1000 mg insbesondere für 10, 11, 12...20, 21, 22, 23..., 28, 29, 30, 31, 32..., 40..., 50..., 67..., 70..., 80...., 100...,110..., 130..., 150, 151, 152..., 170 ,200..., 250..., 280..., 320..., 350..., 400..., 450..., 500, 501, 502, 503..., 556, 557..., 600..., 640..., 680..., 730..., 780..., 823..., 856..., 912..., 954, 955..., 980..., 1000. Entsprechendes gilt für die engeren Mengenbereiche.

Der klinische Erfolg der Bekämpfung der Proteinverlust-Gastroenteropathie durch die Verabreichung von Mannose konnte beispielsweise an einem männlichen, jugendlichen Patienten gezeigt werden, der mit einem normalen Geburtsgewicht geboren worden war. im Alter von 11 Monaten zeigten sich dann klinische Symptome wie Durchfall und Übergeben. Das Hauptsymptom in den folgenden Jahren war die Proteinverlust-Enteropathie. Zusätzlich traten häufig Thrombosen auf.

Im Alter von 6 Jahren traten bei dem Patienten dann schwere gastrointestinale Blutungen auf und führten zu einem lebensbedrohenden Zustand. Zu diesem Zeitpunkt wurde dann begonnen, Mannose oral in einer Anfangsdosis von 100 mg/kg dreimal pro Tag zu verabreichen. Acht Monate später wurde die Dosis auf 150 mg/kg fünfmal pro Tag erhöht. Die gastrointestinalen Blutungen und die chronische Diarrhö verschwanden innerhalb der ersten Wochen. Im Laufe der Therapie verschwanden die Symptome vollständig.

## Patentansprüche

1. Verwendung von Mannose und/oder eines mannosehaltigen Zuckers oder mehrerer mannosehaltiger Zucker, aus dem bzw. denen Mannose im Magen-Darm-Trakt freigesetzt wird, zur Herstellung eines Mittels zur Bekämpfung der Proteinverlust-Enteropathie.

2. Verwendung nach Anspruch 1.
**dadurch gekennzeichnet,**
**daß** das Mittel eine Einzeldosis darstellt und 0,2 g bis 30 g Mannose oder Mannoseeinheiten enthält.

3. Verwendung nach Anspruch 2,
**dadurch gekennzeichnet,**
**daß** das Mittel 2 bis 20 g Mannose oder Mannoseinheiten enthält.

4. Verwendung nach einem der vorhergehenden Ansprüche,
**dadurch gekennzeichnet,**
**daß** die Mannose und/oder der mannosehaltige Zucker oder die mannosehaltigen Zucker in Wasser gelöst ist bzw. sind.

5. Verwendung nach einem der vorhergehenden Ansprüche,
**dadurch gekennzeichnet,**
**daß** es sich bei dem Mittel um ein Nahrungsmittel oder ein pharmazeutisches Mittel handelt.

6. Verwendung nach einem der vorhergehenden Ansprüche,
**dadurch gekennzeichnet,**
**daß** die Mannose und/oder der mannosehaltige Zucker oder die mannosehaltigen Zucker in einer solchen Menge eingesetzt werden, daß 10 bis 100 mg Mannose oder Mannoseeinheiten pro kg Körpergewicht und pro Tag zur Verfügung stehen.

## Claims

1. The use of mannose and/or of a mannose-containing sugar or several mannose-containing sugars from which mannose is released in the gastro-intestinal tract, for producing a preparation for combating protein loss enteropathy.

2. The use according to claim 1, **characterized in that** said preparation is a single dose and contains 0.2 g to 30 g of mannose or mannose units.

3. The use according to claim 2, **characterized in that** said preparation contains 2 to 20 g of mannose or mannose units.

4. The use according to any one of the preceding claims, **characterized in that** the mannose and/or the mannose-containing sugar or the mannose-containing sugars is/are dissolved in water.

5. The use according to any one of the preceding claims, **characterized in that** said preparation is a food product or a pharmaceutical drug.

6. The use according to any one of the preceding claims, **characterized in that** the mannose and/or the mannose-containing sugar or the mannose-containing sugars is/are used in an amount so that 10 to 100 mg of mannose or mannose units is available per kg of body weight and per day.

## Revendications

1. Utilisation de mannose et/ou d'un sucre contenant du mannose ou de plusieurs sucres contenant du mannose dont le mannose est libéré dans le tract gastro-intestinal pour la fabrication d'un moyen pour combattre l'entéropathie par perte de protéines.

2. Utilisation selon la revendication 1, **caractérisé en ce que** le moyen consiste en une dose individuelle et contient de 0,2 g à 30 g de mannose ou d'unités de mannose.

3. Utilisation selon la revendication 2, **caractérisé en ce que** le moyen contient de 2 à 20 g de mannose ou d'unités de mannose.

4. Utilisation selon l'une quelconque des revendications précédentes, **caractérisé en ce que** le mannose et/ou le sucre contenant du mannose ou les sucres contenant du mannose est, respectivement, sont, dissout dans l'eau.

5. Utilisation selon l'une quelconque des revendications précédentes, **caractérisé en ce que** le moyen concerné est un moyen nourricier ou un moyen pharmaceutique.

6. Utilisation selon l'une quelconque des revendications précédentes, **caractérisé en ce que** le mannose et/ou le sucre contentant du mannose ou les sucres contenant du mannose sont mis en oeuvre en une quantité telle que 10 à 100 mg de mannose ou d'unités de mannose par kilogramme de poids corporel et par jour sont disponibles.
